# EUROPEAN PATENT SPECIFICATION

(11) **EP 4 192 444 B1**
(45) Date of publication and mention of the grant of the patent: **12.02.2025**
(21) Application number: 21759264.1
(22) Date of filing: 04.08.2021
(51) Int. Cl.: A61K 31/195, A61K 31/198, A61K 31/661, A61K 31/7008, A61K 31/7084, A61P 25/28

(54) **AMINOACIDS FOR THE TREATMENT OF NEUROLOGICAL DISORDERS**
AMINOSÄURE ZUR BEHANDLUNG VON NEUROLOGISCHEN KRANKHEITEN
ACIDES AMINÉS POUR LE TRAITEMENT DE DÉSORDRES NÉUROLOGIQUES

(30) Priority: 04.08.2020 WO PCT/EP2020/071903
(43) Date of publication of application: 14.06.2023
(73) Proprietor: ScandiBio Therapeutics AB, 171 65 Solna (SE)
(72) Inventor: MARDINOGLU, Adil, 412 60 Göteborg (SE); BORÉN, Jan, 414 67 Göteborg (SE); UHLÉN, Mathias, 181 90 Lidingö (SE)
(74) Representative: Kransell & Wennborg KB
(86) International application number: PCT/EP2021/071785
(87) International publication number: WO 2022/029184

(56) References cited:
- WO-A2-2020/064946
- ADIL MARDINOGLU ET AL: "The Potential Use of Metabolic Cofactors in Treatment of NAFLD", NUTRIENTS, vol. 11, no. 7, 12 July 2019 (2019-07-12), pages 1578, XP055754619, DOI: 10.3390/nu11071578
- RALPH D M ET AL: "Histidine, cystine, glutamine, and threonine collectively protect astrocytes from the toxicity of zinc", FREE RADICAL BIOLOGY & MEDICINE, ELSEVIER INC, US, vol. 49, no. 4, 15 August 2010 (2010-08-15), pages 649 - 657, XP027136603, ISSN: 0891-5849, [retrieved on 20100604]
- ZHANG CHENG ET AL: "The acute effect of metabolic cofactor supplementation: a potential therapeutic strategy against non-alcoholic fatty liver disease", MOLECULAR SYSTEMS BIOLOGY, THE MACMILLAN BUILDING, LONDON, GB, vol. 16, no. 4, 31 March 2020 (2020-03-31), XP009526642, ISSN: 1744-4292, DOI: 10.15252/MSB.209495

## Description

### TECHNICAL FIELD

The invention relates to treatment of neurological disorders.

### BACKGROUND

Neurological disorders such as neurodegenerative diseases (NDDs) are a broad range of complex neurological disorders. The World Health Organization (WHO) estimates that by 2040, NDDs including cognitive-compromising disorders (e.g. Alzheimer's disease (AD), the most common form of dementia) and conditions compromising motor functions (for instance, Parkinson's disease (PD)), will become the second leading cause of death worldwide, taking over cancer after cardiovascular disease. In 2007, the WHO reported that one billion people worldwide were suffering from NDDs, comprising a broad range from AD, PD, multiple sclerosis, epilepsy, brain injuries to neuroinfectious and others. Nowadays, it is estimated that dementia affects 50 million people worldwide, predicting 10 million new dementia cases every year (one every three seconds). The regional distribution of new dementia cases is approaching 4.9 million in Asia, 2.5 million in Europe, 1.7 million in America and 0.8 million in Africa. Out of these figures, 60-70% of the cases are of AD. WHO projected that dementia cases will be raised to 82 million in 2030 and 152 million in 2050. The Global Burden of Disease (GBD) 2016 estimations, based on collected information by the Institute of Health Metrics and Evaluation (IHME), reported that: China, followed by USA, then Japan and India were the countries with highest mortality rates from AD and PD, AD being more frequent in females and PD in males. However, IHME GBD 2016 estimations of global deaths, disability adjusted life years (DALYs) and years lived with disability (YLDs) are significantly higher in AD (2.4 million, 28.8 million, 6.4 million, respectively), compared to PD (211.3 thousand, 3.2 million, 706.4 thousand, respectively). The non-profit group AD International predicted that in 2050, 68% of the people affected with dementia will be from low and middle-income countries. Moreover, the estimated worldwide total cost of dementia was US$ 818 billion in 2015, rising to a trillion dollar in 2018 and predicted to increase to US$ 2 trillion by 2030.

The pathophysiology of NDDs is generally considered to be a complex development with multifactorial aetiologies, where genetic, environmental, and behavioral factors contribute with causal roles. Essential epigenome mechanisms appear to be dysregulated in NDDs. However, such neuroepigenetic modifications are dynamic and to some extent reversible, since they are somatically nonheritable. In PD less than 10% of the diagnosed cases are inherited, with environmental risk factors being a strong component [5, 14]. Similar to PD, most of the AD cases are sporadic. Aging (the greatest risk factor), positive familiar association, head trauma, sex, clinical history of depression, type 2 diabetes mellitus (T2DM), hyperlipidaemia, cerebrovascular conditions, apolipoprotein E4 allele and Down syndrome (DS) are well known risk factors associated to progression of NDDs. Moreover, in the past few years, several studies have been focused on the role of oral and intestinal microbiota and respective metabolites in the development of neurodegenerative diseases, in promoting and also preventing disease progression. Such bidirectional communication between the oral and gut microbiota and the brain in the context of NDDs have been explored.

The exponential rise of cases diagnosed with NDDs has led to the discovery of novel biomarkers that could provide an earlier diagnosis previous to notorious neurological symptoms and identification of novel drug targets that can be used in development of effective drugs. Potential novel therapies may halt or slow down the degeneration processes in the rising burden of NDDs. No pharmacological agent is approved specifically for the effective treatment of AD and PD.

WO2020/064946 discloses a composition that can treat or prevent at least one physical state selected from the group consisting of oxidative stress, a condition associated with oxidative stress, a reduced level of glutathione, and a condition associated with a reduced level of glutathione. The composition contains an effective amount of a combination of at least one glycine or functional derivative thereof, at least one N-acetylcysteine or functional derivative thereof, and at least one nicotinamide riboside or NAD + precursor. The composition can be orally administered, for example as one or more of a food product, a food for special medical purposes (FSMP), a nutritional supplement, a ready to drink formula, a dairy-based drink, a low-volume liquid supplement, powder formats for liquid reconstitution or a meal replacement beverage.

WO2020/7064946 discloses nicotinamide in a list of NAD+ precursors and AD and PD in a list of neurodegenerative diseases. There is no mention of serine or carnitine in WO2020/7064946.

### SUMMARY

NDDs encompass a wide range of progressive neurological disorders and are commonly characterized by specific nerve cells death, but in some cases overlapped clinical manifestations. AD, PD, Frontotemporal lobar degeneration (FTLD), Huntington's disease (HD) and Amyotrophic lateral sclerosis (ALS) are representative NDDs characterized by a shared hallmark: the misfolding, followed by aggregation and deposition of neurotoxic proteins, a unique protein in each disease in specifically distinct anatomic brain regions. Disease symptoms are often related to the respective functional anatomic brain area(s) under degeneration. For instance, the hippocampus (essential for learning and memory) is the area affected in AD at an early stage, then the brain lesions spread with disease progression; while in PD the dopaminergic neurons in the midbrain are the ones suffering primary degeneration. Recent studies have been reporting dysregulation of the brain inflammatory response associated with a state of chronic oxidative stress as a hallmark of NDDs. Different mechanisms triggering the loss of a balanced redox state, through the dysregulation of proinflammatory signaling molecules secretion in the brain environment, have been reported in numerous studies. Under conditions threatening the immune system and homeostasis, certain enzymes (e.g. NOX2, an enzyme of phagocytic and other cells, during exposure to pathogens) produce ROS as part of the inflammatory response. Moreover, stimulus by cytokines, growth factors, hyperglycemia and hyperlipidemia contribute to such enzymatic activity creating an increased concentration of reactive species. Abnormal mitochondria dysfunction due to mutations of specific molecules (e.g. leucine-rich repeat kinase 2 (LRRK2), PINK-1, DJ-1 or α-synuclein, reported in PD), misfolding and aggregation of neurotoxic proteins due to genetic factors and ageing, but also due to brain microbial infection (e.g. by *Candida albicans, Salmonella enterica*) or due to gut produced microbial metabolites that migrate to the brain are studied mechanisms promoting inflammation and subsequently perturbing the brain redox balance. Thus, disease-specific signatures of the neuroinflammatory microenvironment have been identified when compared to the immune landscape steady-state of the central nervous system (CNS). The key role of migration by microglia, astrocytes and peripheral myeloid cells in the mediation of the CNS inflammation and disease progression is further discussed below.

The deposition of Aβ plaques are a well-known hallmark of AD, which has been associated to a state of oxidative stress. Studies have verified an increased activity of Th17 cells in AD, while the secretion of IFN-y by Th1 and IL-17 by Th17 promote hypoactivity of Th2 cells, decreasing its secretion of IL-4 and IL-10, which it is known for its anti-inflammatory activity in AD. Thus, Th2 cells transfer reveals to be a potential therapeutic approach in AD. Regarding regulatory T cells, based on a murine model, these have shown to play a beneficial role in the AD pathophysiology and disease progression. The study implemented a peripheral injection of IL-2 low-dose treatment, which revealed an increased number of protective microglia together with restored cognitive functions in APPPS1 mice. Thus, immunotherapy based on IL-2 peripheral injection for Tregs modulation appears as a promising approach for the delay of disease progression.

A hallmark of PD is the degeneration of dopaminergic neurons located in the substantia nigra. Such neural loss has been linked to a state of redox imbalance with increased number of reactive species, which can be promoted by mitochondrial dysfunction due to mutations of target molecules. It has been demonstrated in animal models that effector T cells express dopamine D3 receptors (D3R), showing that CD4+T cells activated through this mechanism stimulated differentiation into the proinflammatory Th1 phenotype. Thus, it was assumed that D3R plays a key role in the loss of dopaminergic neurons, compromising the substantia nigra. Therefore, D3R can be seen as a potential therapeutic drug target in PD neurodegeneration.

In a previous study, the present inventors have found that the level of GSH is not enough to maintain and regulate the thiol redox status of the liver in subjects with high hepatic steatosis at fasting stage due to the depletion of glycine. Glycine can be synthesized via the interconversion of serine. It was shown that the serine synthesis is downregulated in patients with NAFLD and supplementation of serine has attenuated alcoholic fatty liver by enhancing homocysteine metabolism in mice and rats. Depleted liver GSH is also restored by the administration of N-acetylcysteine as in acetaminophen poising. L-carnitine and nicotinamide riboside (NR) that both stimulate the transfer of fatty acids from cytosol to mitochondria were identified as two additional cofactors that are depleted in patients. The strategy was to enhance the mitochondrial activity by supplementation of (1) L-carnitine to enhance the transport of fatty acids across the mitochondrial membrane, (2) the NAD+ precursor nicotinamide riboside, and the (3) GSH precursors serine and N-acetylcysteine (NAC).

To test the model-based predictions, the present inventors assessed the effect of short-term dietary supplementation with serine on fatty liver and fasting levels of plasma markers of liver functions in six obese subjects (BMI 32.5 ± 2.70 kg/m2) with hepatic steatosis. Each patient received one oral dose of ~20 g of L-serine (200 mg/kg) per day for 14 days. The supplementation was well-tolerated by all subjects. Results showed that ALT, AST and ALP levels were significantly decreased after supplementation, and that the liver fat decreased significantly from 26.8 ± 6.0 % to 20.4 ± 7.0%.

The present inventors examined the concept by testing if supplementation of GSH and NAD⁺ precursors for two weeks would decrease high fat diet induced liver fat accumulation in mice. Therefore, high fat diet-fed mice were given serine (300 mg/kg/day) and nicotinamide riboside (NR) (400 mg/kg/day) *la gavage* as and 1g/l of NAC (N-acetyl-L-cysteine) in the drinking water for 14 days. Analysis of the liver lipids demonstrated a 50% reduction in hepatic triglycerides and a tendency to decreased levels of cholesterol esters. The chain lengths of the hepatic triglycerides were also analysed and it was found that shorter chain lengths of triglycerides (that are preferentially oxidized in the mitochondria) were significantly decreased after the supplementation. Hence, it was demonstrated that supplementation of GSH and NAD+ precursors (i.e., the metabolites that were predicted to promote oxidation of fat in the liver), prevented hepatic steatosis in high fat diet-fed mice.

Chronic loss of a balanced redox state by dysregulation of inflammatory response, culminates in exacerbated neuroinflammation, which is considered as a hallmark of NDDs. In such a chronic oxidative environment, the antioxidant defense systems responsible for modulating the proinflammatory responses are dysregulated by increased signaling molecules such as reactive oxygen species (ROS), which promote activation of phosphorylation pathways while inhibiting dephosphorylation enzymes. Consequently, the dysregulation of cellular transduction signals enhances the secretion of proinflammatory molecules (e.g. cytokines and chemokines), neoepitopes and production of danger-associated molecular patterns (DAMPs). Thus, in a state of oxidative stress, the increased concentrations of reactive species (e.g. ROS, reactive nitrogen species (RNS) and free radicals) contribute to the stimulation and activation of microglia and astrocytes. Such neuro-proinflammatory response promotes the recruitment of CD4+T cells to the CNS under inflammatory conditions, which in turn intensify the activation of microglia and astrocytes, generating a vicious cycle. The exacerbation of such a cascade of mechanisms is capable of perpetuating the proinflammatory response leading to the loss of the regulation of the neuroinflammation response observed in NDDs.

Reactive species act as signaling molecules in cellular metabolic pathways and physiological functions, contributing to homeostasis, while being modulators of the inflammatory response. In a chronic state of oxidative stress, the effects of an imbalanced number of endogenous and exogenous reactive species creates a damaging state with adverse consequences (e.g. protein/lipid ionization; DNA mutations). Continuously, the chronic loss of redox balance leads to the stimulation of signaling pathways involved in the secretion of proinflammatory molecules, such as IL-6, IL-11, interferons (IFNs) and tumor necrosis factor (TNF) promoting the production of reactive species, thus reinforcing the activation of a proinflammatory loop. In the case of NDDs, chronic oxidative stress promotes a chronic neuroinflammatory environment, with subsequent activation of microglia and astrocytes (accounting for 10% and 20-30% of the glial cells of the human brain, respectively) and infiltration of peripheral myeloid cells into the CNS, that in the long term leads to progressive tissue damage culminating in neurodegeneration. Naturally, with disease progression the phenotype pool of recruited CD4+T cells into the CNS becomes more diverse, being accompanied by phenotypical changes of microglial cells that lose their quiescent state. In the case of AD, as previously mentioned, an imbalanced oxidative state is associated with Aβ plaques deposition. This association leaded the present inventors to the understanding that: initially, tau protein hyperphosphorylation is promoted by the activation of p38 MAPK signaling pathway, subsequently leading to neurofibrillary tangles formation; followed the mitochondrial release of cytochrome C, which is triggered by death the promoter Bcl-2 that mediates apoptotic pathways. On a later stage, such neuroinflammatory mechanism culminates with the recruitment of T cells into the brain parenchyma.

Insulin resistance promotes several metabolic abnormalities, such as hyperglycaemia (a main feature of T2DM), hyperlipidemia, inflammation and oxidative stress. Memory, cognition, emotional functions and the central modulation of body metabolism are regulated by the neuronal role of insulin. Therefore, molecular signaling pathways mediated by insulin have significant impact on brain function. Consequently, impairments in the synaptic neurotransmission, metabolic and neuroinflammatory immune responses can be stimulated by brain insulin resistance. Thus, a connection between T2DM, brain insulin resistance and AD development have been supported by epidemiological, clinical and animal studies. Considering the nature of such connections, based on the shared underlying cellular and molecular mechanisms, it has been suggested a repurpose of anti-diabetic drugs in the treatment of brain insulin resistance under AD circumstances. Moreover, obesity-linked insulin resistance has been considered as one of the main causes of T2DM. Additionally, fatty liver, cardiovascular diseases and cancer are also known to be promoted by obesity. A chronic low-level of inflammation underlies several metabolic disorders including obesity and diabetes, which in these cases is mediated by the secretion of interleukins and TNF-α. Thus, there is an emerging interest in understanding shared molecular mechanisms linking these metabolic disorders (e.g. T2DM) in order to prevent and slow down AD progression, as well as in the context of other NDDs.

Systems biology facilitates this hurdle by enabling integration and analysis of multi-layer omics data not only to understand the pathophysiology, but also to make predictions that can be experimentally tested. This integrative concept has successfully been used in a variety of contexts in medical research during the last decade. Along this concept, the present inventors have in a series of studies combined clinical studies with stable isotopes, systems medicine and in-depth multi-omics profiling to clarify the underlying mechanisms of NDDs with the aim of developing strategies for prevention and treatment.

It has been observed that altered nicotinamide adenine dinucleotide (NAD+) and glutathione (GSH) metabolism (with increased demand for NAD and GSH) is a prevailing feature in NDDs, and that subjects with NDDs have reduced *de novo* synthesis of GSH. The plasma level of glutathione (GSH) is also typically depleted in individuals with metabolism related disorders. However, cellular GSH levels cannot be increased by supplementing GSH and it must be synthesized within the human body either *de novo* or by the salvation pathway.

As further described below under Examples, the present inventors have examined the above concept by testing if supplementation of GSH and NAD+ precursors improves the cognitive functions in Parkinson's mouse treatment models. The testing demonstrated that the supplementation improved the health status of the Parkinson's mice models.

The precursors/substances of the testing were serine, carnitine, NR and N-acetyl-L-cysteine (NAC).

Considering metabolic pathways, the present inventors have identified the following alternatives to the above-mentioned substances:

| Substance | Alternatives |
|---|---|
| serine | glycine, betaine, N-acetylglycine, N-acetylserine, dimethylglycine, sarcosine and/or phosphoserine |
| | |
| NAC | Cysteine and/or cystine |
| | |
| L-carnitine | deoxycarnitine, gamma-butyrobetaine, 4-trimethylammoniobutanal, 3-hydroxy-N6,N6,N6-trimethyl-L-lysine, N6,N6,N6-trimethyl-L-lysine and/or lysine |
| | |
| NR | quinolinate, deamino-NAD+, nicotinate D-ribonucleotide, nicotinamide D-ribonucleotide, nicotinate D-ribonucleoside, nicotinamide and/or nicotinate |

Accordingly, the present disclosure provides a composition for use in a therapeutic method of treatment of a subject suffering from Alzheimer's disease or Parkinson's disease, said composition comprising:
A) serine;
B) N-acetyl cysteine, cysteine and/or cystine;
C) carnitine; and
D) nicotinamide.

Also, the present disclosure provides substances comprising
A) serine,
B) N-acetyl cysteine, cysteine and/or cystine,
C) carnitine and
D) nicotinamide
for simultaneous, separate or sequential use in a method of treatment of a subject suffering from Alzheimer's disease or Parkinson's disease.

### BRIEF DESCRIPTION OF THE FIGURES

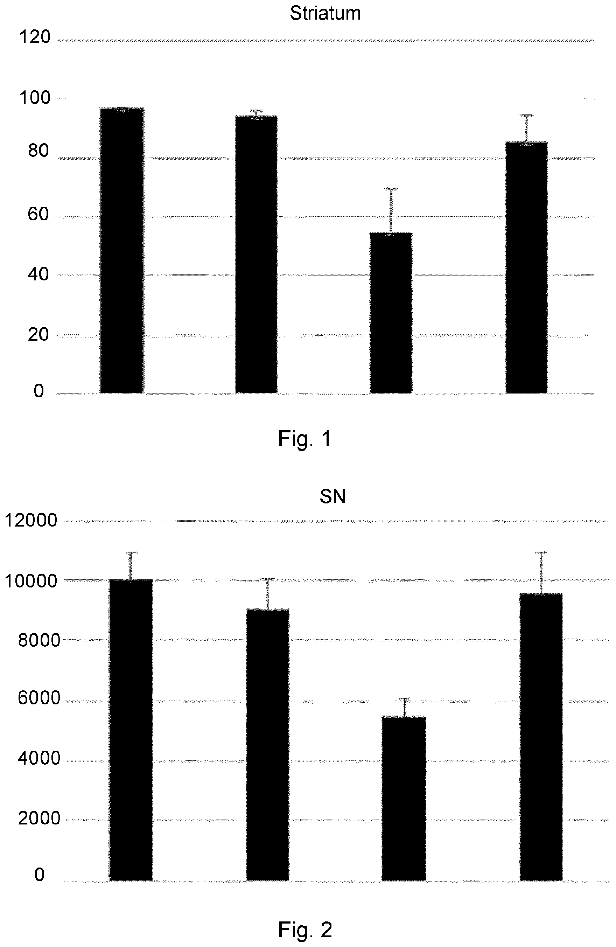

Fig. 1 shows the density of tyrosine hydroxylase-positive fibers in the striatum. The first bar from the left represents Group 1 ("Saline"). The second bar from the left represent Group 2 ("Saline + supplements"). The third bar from the left represents Group 3 ("MPTP"), i.e. the untreated group of mice having MPTP-induced PD. The fourth bar from the left represents Group 4 ("MPTP + supplements"), i.e. the mice having MPTP-induced PD that were given the supplements.
Fig. 2 shows the number of tyrosine hydroxylase-positive cells in substantia nigra (SN). The first bar from the left represents Group 1 ("Saline"). The second bar from the left represent Group 2 ("Saline + supplements"). The third bar from the left represents Group 3 ("MPTP"), i.e. the untreated group of mice having MPTP-induced PD. The fourth bar from the left represents Group 4 ("MPTP + supplements"), i.e. the mice having MPTP-induced PD that were given the supplements.

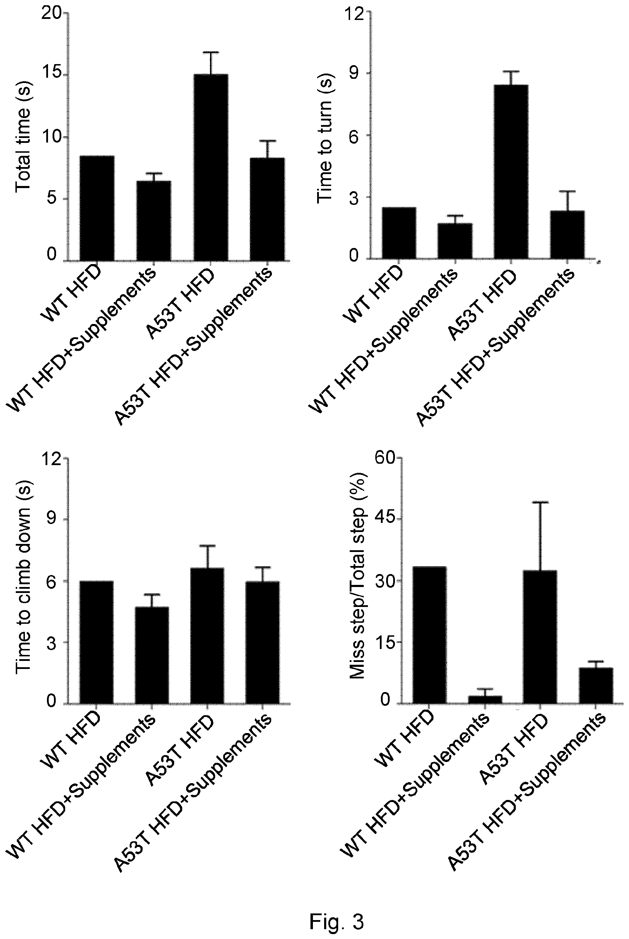

Fig. 3 shows the results of a vertical grid test. In each chart of fig. 3: the first bar from the left represents a wild type mouse; the second bar from the left represent wild type mice given the supplements; the third bar from the left represents untreated virus-induced PD mice; and the fourth bar from the left represents virus-induced PD mice given the supplements.

### DETAILED DESCRIPTION

As a first aspect of the present disclosure, there is provided a composition for use in a method of treating a subject suffering from a neurological disorder. The subject of the present disclosure is preferably a human subject.

The composition comprises:
A) serine;
B) N-acetyl cysteine, cysteine and/or cystine;
C) carnitine; and
D) nicotinamide.

The substance A) is serine, which is usually provided as L-serine.

In group B), N-acetyl cysteine (NAC) and cysteine are preferred. The most preferred substance in group B) is NAC.

The substance C) is carnitine, optionally in the form of a carnitine salt, such as carnitine tartrate. Most preferably, the substance C) is L-carnitine, optionally in the form of a L-carnitine salt, such as L-carnitine tartrate.

The substance D) is nicotinamide.

The substance A) is preferably included in a higher molar amount than the substance D). When efficacy and toxicity is also considered (see the discussion about doses below), the molar ratio of A) to D) is normally between 250:1 and 1.5:1 and typically between 150:1 and 3:1. Preferably, the molar ration is between 90:1 and 10:1, more preferably between 50:1 and 20:1.

In embodiments, the molar ratio of A) to B), considering efficacy and toxicity, is typically between 16:1 and 1:4, preferably between 12:1 and 1.5:1 and more preferably between 10:1 and 3:1.

In embodiments, the molar ratio of A) to C), considering efficacy and toxicity, is normally between 150:1 and 1:1, typically between 100:1 and 2:1, preferably between 30:1 and 3:1, more preferably between 15:1 and 4:1.

The above ratios entail that a patient consuming the composition can obtain appropriate doses of the respective substances.

In one embodiment, the composition of the first aspect is a solid, such as a solid powder. Such a powder can be mixed with water, e.g. by the patient/consumer, a nurse or a physician. In another embodiment, the composition of the first aspect is an aqueous solution or suspension ("cocktail"), which facilitates convenient oral administration. Such an aqueous solution or suspension is preferably ready to drink.

As a particularly preferred embodiment of the first aspect, the composition is a powder comprising:
A) serine;
B) N-acetyl cysteine;
C) carnitine; and
D) nicotinamide, wherein

the molar ratio of A) to B) is between 12:1 and 1:1.5, preferably between 10:1 and 3:1,
the molar ratio of A) to C) is between 100:1 and 2:1, preferably between 30:1 and 3:1, more preferably between 15:1 and 4:1; and
the molar ratio of A) to D) is between 150:1 and 3:1.

As another particularly preferred embodiment of the first aspect, the composition is a powder comprising:
A) serine;
B) N-acetyl cysteine and/or cysteine;
C) carnitine; and
D) nicotinamide, wherein
the molar ratio of A) to D) is between 150:1 and 3:1.

In embodiments of the solution or suspension according to the first aspect:
- the concentration of A) is typically 0.20-2.4 mmol/ml, preferably 0.40-2.4 mmol/ml and more preferably 0.60-2.4 mmol/ml; and/or
- the concentration of D) is typically 0.006-0.12 mmol/ml, preferably 0.012-0.08 mmol/ml and more preferably 0.018-0.07 mmol/ml. In the solution or suspension according to the first aspect:
- the concentration of B) is normally 0.09-0.90 mmol/ml, typically 0.09-0.54 mmol/ml, preferably 0.11-0.40 mmol/ml and more preferably 0.013-0.30 mmol/ml; and/or
- the concentration of C) is normally 0.009-0.38 mmol/ml, typically 0.009-0.19 mmol/ml, preferably 0.016-0.16 mmol/ml and more preferably 0.028-0.12 mmol/ml.

The solution or suspension of the first aspect may be provided in a package for convenient handling and distribution. Further, the volume of such a package may be such that drinking the whole contents of the package at once or during a single day results in oral administration of appropriate doses of the substances in the solution or suspension. In one embodiment, the volume of the package is 25-1000 ml. The volume is preferably 50-500 ml. When it is intended that the consumer/patient shall drink more than one package per day, the volume is typically relatively low, such as 25-500 ml, preferably 25-400 ml.

In one embodiment, the packaged solution or suspension comprises 48-478 mmol of A). Thereby, the dose of A) is effective, but not toxic. In a preferred embodiment, A) is serine in an amount of 5-50 g, more preferably 10-50 g.

In an alternative of complimentary embodiment, the packaged solution or suspension comprises 2.0-196 mmol of D). Thereby, the dose of D) is effective, but not toxic.

When the composition of the first aspect is a powder, it may also be packaged. As an example, the powder may be provided in unit dose packs. It follows from the discussion above that such a unit dose may comprise 48-478 mmol of A) and/or 2.0-196 mmol of D). Further, such as packed powder preferably comprises serine in an amount of 5-50 g. More preferably, such a packed powder comprises serine in an amount of 10-50 g.

The substances of the present disclosure are preferably a significant part of the composition of the first aspect. For example, the substances included in groups A)-D) may amount to at least 10 %, such as at least 25 %, such as at least 50 % of the dry weight of the composition of the first aspect. In one embodiment, the weight of serine is at least 10 %, such as at least 25 %, such as at least 40 % of the dry weight of the composition of the first aspect.

The composition of the first aspect may comprise one or more tasting agent(s), such as one or more sweetener(s) (e.g. sucralose) and/or one or more flavor agent(s). It may also comprise a lubricant, such as a polyethylene glycol lubricant (e.g. Polyglykol 8000 PF (Clariant)).

The neurological disorder treated according to the first aspect is Alzheimer's disease or Parkinson's disease.

In an embodiment, the method of treatment comprises oral administration of the composition.

The method of treatment may for example comprise administration, such as oral administration, of:
A) in a dose of 0.48-24 mmol/kg/day, such as 0.48-4.8 mmol/kg/day, such as 1.8-4.8 mmol/kg/day, such as 2.9-4.7 mmol/kg/day;
optionally B) in a dose of 0.31-3.05 mmol/kg/day, such as 0.31-1.84 mmol/kg/day, such as 0.40-1.23 mmol/kg/day;
optionally C) in a dose of 0.100-2.50 mmol/kg/day, such as 0.200-2.00 mmol/kg/day, such as 0.230-1.00 mmol/kg/day, such as 0.300-0.800 mmol/kg/day; and/or
D) in a dose of 0.020-1.96 mmol/kg/day, such as 0.020-0.39 mmol/kg/day, such as 0.039-0.31 mmol/kg/day, such as 0.059-0.24 mmol/kg/day.

If the doses are not adjusted to the weight of the patient, the method of treatment may for example comprise administration, such as oral administration, of:
A) in a dose of 100-600 mmol/day, such as 150-450 mmol/day, such as 170-350 mmol/day;
optionally B) in a dose of 12-100 mmol/kg/day, such as 16-75 mmol/day, such as 20-50 mmol/day;
optionally C) in a dose of 12-100 mmol/kg/day, such as 16-75 mmol/day, such as 20-50 mmol/day;
D) in a dose of 3-20 mmol/day, such as 4-15 mmol/day, such as 5-12 mmol/day.

The daily dose may be reached by administrating one or more doses per day to the patient. In one embodiment, the number of daily doses is one, two or three. For example, the patient may consume a drink formed from the composition in powderous form one, two or three times per day. Each dose or drink preferably comprises no more than 4.78 mmol/kg of A).

The method of treatment may be carried out for a period of 1 week to 1.5 years, e.g. 1-52 weeks, such as 3-46 weeks, such as 6-30 weeks.

As a second, non-claimed, aspect of the present disclosure, there is provided a method of treatment of a subject suffering from a neurological disorder, comprising administration, such as oral administration, of:
A) serine, for example in a dose of 0.48-24 mmol/kg/day, such as 0.48-4.8 mmol/kg/day, such as 1.8-4.8 mmol/kg/day, such as 2.9-4.7 mmol/kg/day;
B) N-acetyl cysteine, cysteine and/or cystine, for example in a dose of 0.31-3.05 mmol/kg/day, such as 0.31-1.84 mmol/kg/day, such as 0.40-1.23 mmol/kg/day;
C) carnitine, for example in a dose of 0.100-2.50 mmol/kg/day, such as 0.200-2.00 mmol/kg/day, such as 0.230-1.00 mmol/kg/day, such as 0.300-0.800 mmol/kg/day; and
D) nicotinamide, for example in in a dose of 0.020-1.96 mmol/kg/day, such as 0.020-0.39 mmol/kg/day, such as 0.039-0.31 mmol/kg/day, such as 0.059-0.24 mmol/kg/day.

The embodiments and examples of the first aspect apply to the second aspect *mutatis mutandis.*

For the patient/consumer, it is not necessary to take the substances of the present disclosure simultaneously. A therapeutic effect can also be achieved if the substances are taken separately or sequentially, preferably within a day and more preferably within an hour.

As a third aspect of the present disclosure, there is thus provided substances comprising:
A) serine;
B) N-acetyl cysteine, cysteine and/or cysteine;
C) carnitine; and
D) nicotinamide
for simultaneous, separate or sequential use in a method of treatment of a subject suffering from Alzheimer's disease or Parkinson's disease.

The third aspect may for example be a combined preparation of two or more units, such as a first unit comprising A), a second unit comprising D), optionally a third unit comprising B) and optionally a fourth unit comprising C).

The embodiments and examples of the first and second aspect apply to the third *mutatis mutandis.*

### ANIMAL EXPERIMENTS (COMPARATIVE)

**The effect of supplements in a MPTP-based experiment**

### Administration of MPTP and supplements

This experiment was based on the Protocol for the MPTP Mouse Model of Parkinson's Disease (PD) (Nat Protoc. 2007;2(1):141-51; https://pubmed.nebi.nlm.nih.gov/17401348/).

Male C57Bl/6 mice (21-25 g; age 8 weeks) were divided into four groups of three animals each (N=3):
Group 1 ("Saline");
Group 2 ("Saline + supplements");
Group 3 ("MPTP"); and
Group 4 ("MPTP + supplements").

The mice of groups 3 and 4 received intraperitoneal injections of MPTP. The mice of groups 1 and 2 received intraperitoneal injections of sterile saline. MPTP was administered as 4 times injections of 20 mg/kg at 2 hr intervals. Control animals were injected an equal volume of 0.9% sterile saline.

The supplements were:
serine (400 mg/kg/day *la gavage);*
carnitine tartrate (400 mg/kg/day *la gavage);*
NR (400 mg/kg/day *la gavage);* and
NAC (1g/l in the drinking water to a total of 400 mg/kg/day).

The supplements were given once a day starting day 4 after the final MPTP injection. The control mice received water on day 3 after the final MPTP injection. The mice were sacrificed seven days after the final MPTP injection.

### Immunohistochemistry and results

Since PD is generally considered to a tyrosine hydroxylase-deficiency syndrome, the tyrosine hydroxylase level in two parts of the brains of the mice was measured.

The sacrificed mice were perfused with a perfusion solution and post-fixated with 4% paraformaldehyde for one day at 4°C. The perfusion solution contained NaCl, NaNO₃, (Sigma-Aldrich) and heparin dissolved in distilled water. The perfused mice were stored in a 30% sucrose solution for 24-48h at 4°C until they sank. Then, the brains of the mice were cut coronally to slices of 30 µm thickness. Brain slices were fixed with tissue stock solution and rinsed three times in Phosphate-buffered saline (PBS pH 7.4). 0.3% TritonX-100, 2% donkey serum (Gene Tex, Irvine, CA, USA) in PBS was used for blocking for 90 min, then brain slices were contacted with anti-tyrosine hydroxylase at 4°C for overnight. After that, brain slices were incubated with rabbit secondary antibodies (Dako EnVision⁺ system-HRP, USA) for 90 min, and then reacted with DAB⁺ substrate buffer. After mounting by fluorescent mounting medium (Dako North America Inc, USA) on cover slides, immunofluorescent images were acquired using Olympus^{™} confocal microscope (Olympus).

The density of tyrosine hydroxylase-positive fibers in the striatum was quantified. Group 4 showed a significantly higher density than Group 3 (see Fig. 1). In other words, the supplements significantly increased the tyrosine hydroxylase level in mice having MPTP-induced PD. No corresponding effect was observed in mice without MPTP-induced PD (compare Group 2 to Group 1). Notably, the tyrosine hydroxylase level in the supplemented mice having an MPTP-induced PD (i.e. Group 4) was almost as high as in the control group (i.e. Group 1).

Further, the number of tyrosine hydroxylase-positive cells in substantia nigra (SN) was quantified. Group 4 showed a significantly higher number than Group 3 (see Fig. 2). In other words, the supplements significantly increased the tyrosine hydroxylase level in mice having MPTP-induced PD also in the SN. No corresponding effect was observed when Group 2 was compared to Group 1. Notably, the tyrosine hydroxylase level in the supplemented mice having an MPTP-induced PD (i.e. Group 4) was as high as in the control group (i.e. Group 1).

In conclusion, the experiment supports that the supplements are effective in treating neurological disorders, such as PD.

**The effect of supplements in a virus injection mouse model**

C57BL/6J male mice were used for the experiment. When the experiment started, the mice were 8 weeks old. The mice, which were given a high fat diet throughout the experiment, were divided into four groups.
Group 1 (N=1): wild type mouse
Group 2 (N=3): wild type mice, administration of supplements starting on day 15
Group 3 (N=3): virus-induced PD mice
Group 4 (N=3): virus-induced PD mice, administration of supplements starting on day 15

Virus-induced PD was achieved by stereotaxic injections of alpha-synuclein (AAV-A53T virus) in the substantia nigra of the 8 weeks old mice of groups 3 and 4.

The supplements were:
serine (400 mg/kg/day *la gavage);*
carnitine tartrate (400 mg/kg/day *la gavage);*
NR (400 mg/kg/day *la gavage);* and
NAC (1g/l in the drinking water to a total of 400 mg/kg/day).

The supplements were dosed daily. After the 15^{th} dose, the mice were fasted for 4 hours and then, the vertical grid test was performed.

The apparatus for the vertical grid test was an open box (8 cm × 55 cm × 5 cm) with a black wall made of 0.8 cm × 0.8 cm wire mesh. All groups were adapted to the vertical grid test by placing inside the apparatus for 5 days and allowing them to turn and climb down. Experiments were performed by recording and analysing total time, time to turn, time to climb down and missteps/total steps.

The vertical grid test showed that the supplements significantly reduced the total time, the time to turn and the proportion of missteps of group 4 compared to group 3 (Fig. 3). This supports that the supplements are effective in treating neurological disorders, such as PD.

### CLINICAL TRIAL (HUMANS) (COMPARATIVE)

### Trial design

### Preparation of the investigational drug and placebo

This trial is a double-blind, randomized, placebo-controlled, investigator-initiated, multi-centre trial, which aims to establish metabolic improvements in AD and PD subjects by dietary supplementation with N-acetylcysteine, L-carnitine tartrate, nicotinamide riboside and serine. In the trial, supplements or matching placebo are taken by the subjects as powder dissolved in water by mouth.

The drug product, i.e. the mixture of the four supplements, and the placebo was produced in Turkey according to GMP regulations by Pharmactive Company (www.pharmactive.com.tr/en/anasayfa.html) according to EU standards and packed according to GMP rules with the appropriate dosage.

The drug product and the placebo were not identifiable from its packaging. Both were dissolved by patients in 200 mL preferably cold water before use.

The drug product and the placebo were administered in white plastic bottles (containing dry powder) with a desiccant cap.

Stability tests performed by ChromaDex in USA has shown that the mixture of supplements is stable at 25°C for at least three months in the plastic bottles.

Strawberry aroma had been added to the drug product. As placebo, sorbitol (5 g) flavoured with strawberry aroma and colouring agent were given.

### Study population

The study population was 69 Alzheimer's disease and 48 Parkinson's disease patients on Day 0 and Day 28; 60 Alzheimer's disease and 43 Parkinson's disease patients on Day 84. Eligible subjects had signed an informed consent, met all inclusion criteria and had none of the exclusion criteria listed below.

### Inclusion criteria

I-1. Men and women diagnosed with Parkinson's Disease (Hoehn Yahr 2-4, age >40 years) or men and women diagnosed with Alzheimer's Disease. Include patients older than 50 years with mild to moderate Alzheimer's disease according to ADAS-cog (Alzheimer's Disease Assessment Scale-cognitive subscale; ADAS≥12) and the Clinical Dementia Rating Scale Sum of Boxes (CDR-SOB; CDR≤2).

I-2. Patients with stable treatments and clinical course

### Exclusion criteria

E-1. Inability or unwillingness to give written informed consent
E-2. History of stroke, severe brain trauma, toxic drug exposure
E-3. Neurological examination which indicate to Parkinson-Plus syndrome (i.e., pyramidal, cerebellar and autonomic dysfunction findings and gaze paralysis) for PD
E-4. Uncontrolled Type 1 or type 2 diabetes
E-5. Diarrhea (defined as more than 2 stool per day) within 7 days before enrolment
E-6. Chronic kidney disease with an estimated glomerular filtration rate <60 ml/min/1.73m2
E-7. Significant cardiovascular co-morbidity (i.e. heart failure)
E-8. Patients with active bronchial asthma
E-9. Patients with phenylketonuria (contraindicated for NAC)
E-10. Patients with histamine intolerance
E-11. Clinically significant TSH level outside the normal range (0.04-6 mU/L)
E-12. Known allergy for substances used in the study
E-13. Concomitant medication use:
   o Self-administration of dietary supplements such as any vitamins, omega-3 products, or plant stanol/sterol products within 1 week
   o Use of an antimicrobial agent in the 4 weeks preceding randomization
E-14. Active smokers consuming >10 cigarettes/day
E-15. Alcohol consumption over 192 grams for men and 128 grams for women per week
E-16. Patients considered as inappropriate for this study for any reason (patients unable to undergo MRI study, noncompliance etc.)
E-17. Active participation in another clinical study
E-18. Women with potential to give birth. Post-menopausal women, women who had hysterectomy or women with acceptable medical proof shoving that she can't get pregnant according to the investigator's decision may be enrolled to the study.

### Randomization and blinding

The study subjects were randomized on a 2:1 basis to the supplements mixture or placebo. A web-based randomization system was used to assign a randomization code for each patient. An investigator or other responsible person at the investigational site was able to enter the web-based randomization system specific to the study through assigned username and password. After entering patient-related information (patient number, date of birth, patient initials), the system provided randomization code for the future use by investigators. This randomization code was entered into the electronic case report form (e-CRF).

### Dosage and administration

Subjects in active treatment received dietary supplementation with N-acetylcysteine, L-carnitine tartrate, nicotinamide riboside, and serine, administered as a mixture. Half dosage of the supplements was given for four weeks (one dose taken just after dinner), and full dosage for 8 weeks (two equal doses taken just after breakfast and dinner).

The dosage of the supplements will be as follows:

| | **Weeks 1-4 (28 days)** | **Weeks 5-12 (56 days)** |
|---|---|---|
| L-Carnitine tartrate | 3.73 g/day | 7.46 g/day |
| N-Acetylcysteine | 2.55/day | 5.1 g/day |
| Nicotinamide riboside | 1 g/day | 2 g/day |
| Serine | 12.35 g/day | 24.7 g/day |

### Study plan

The study comprises four clinical visits.

### Visit 1

Screening visit (Weeks -1; Days -3 to -1). The subjects got the informed consent form and oral information on the study protocol, including genetic studies, number of visits and procedures to be performed in each visit. After the subject had given written informed consent to participate in the study, the data obtained in the screening visit included the following:
- Demographic data
- Medical history including associated conditions, previous operations, smoking and alcohol consumption and concomitant drug usage
- Physical examination including body weight, height, waist circumference, hip circumference, blood pressure and pulse rate
- Neurological examination to stage PD and AD patients
- Functional Medicine Assessment
- Review of inclusion/exclusion criteria
- Laboratory safety parameters including complete blood count (blood cells, haemoglobin), alkaline phosphatase (ALP), alanine aminotransferase (ALT), Aspartate aminotransferase (AST), gamma-glutamyl transferase (GGT), bilirubin, albumin, creatinine kinase (CK), total cholesterol, high density lipoprotein-cholesterol (HDL-C), low density lipoprotein-cholesterol (LDL-C), triglycerides, creatinine, urea, urate, glucose, sodium (Na), potassium (K), insulin, HbA1c and thyroid-stimulating hormone (TSH)
- The motor and cognitive functions of PD patients evaluated via UPDRS and MoCa, respectively.
- The cognitive functions of AD patients evaluated via MMSE and ADAS-cog.
- The daily life activity of AD patients evaluated via ADCS-ADL.
- Clinical Dementia Rating Scale Sum of Boxes (CDR) for clinical evaluation of AD.
- The behavioral functions of PD and AD patients will be evaluated via NPI.
- CNS atrophy and functional network activity of PD and AD patients evaluated via volumetric Magnetic resonance Imaging (MRI) and resting state functional magnetic resonance imaging (rest-fMRI), respectively.
- Information on prior and concomitant medications will be collected

### Visit 2

Randomization visit (Week 1; Day 1). Eligible study subjects were randomized to active therapy or placebo groups and study agents were dispensed.

At the randomization visit the subjects were asked to take the first dose at the hospital and observed for development of adverse events. The changes in concomitant medications were evaluated. Patients who tolerated the study agents started to take half dose of supplements mixture (i.e., one administration daily just after dinner) for four weeks. Treatment compliance and adverse event questioning were performed by the study nurse by weekly telephone contacts.

### Visit 3

On-treatment first visit (Week 4; Day 28). The subjects came to the study center for complete evaluation including and adverse events recording. Clinical and physical examination, determination of the motor, cognitive and behavioral functions using clinical scales, laboratory safety parameters, lipidomic, metabolomic and oral/gut microbiota analysis were repeated as in Visit 1.

Clinical scales included MMSE, ADAS-cog, ADCS-ADL and CDR for AD patients and UPDRS and MoCA for PD patients. NPI was applied to both group of patients in order to evaluate behavioral symptoms.

After Visit 3, subjects started to take full dose of final concentrations (i.e., two dosages daily taken just after breakfast and after dinner). Treatment compliance and adverse event questioning were performed by the study nurse by weekly telephone contacts.

### Visit 4

End of study, on-treatment second visit (Week 12; Day 84). The subjects returned to the study center after eight weeks to evaluate efficacy, tolerability and safety.

At the end of the treatment, all procedures including clinical and physical examination, adverse events recording, MRI volumetric and rest-state fMRI, determination of the motor, cognitive and behavioral functions using clinical scales, biochemical, lipidomic, metabolomic and oral/gut microbiota analysis were repeated as in Visit 1. Treatment compliance and development of any adverse events were questioned during this visit.

After Visit 4, participants stopped taking study agents.

### Results

The present disclosure can present the MoCA results at Day 28 for 32 PD subjects given the drug product and 16 PD subjects given placebo (i.e. results from Visit 3). In treated group, there was a significant positive effect (p-value: 0.0013; log2foldchange: 0.173). There was also a significant effect in the group given placebo (p-value: 0.001; log2foldchange: 0.159).

Further, the present disclosure can present the MoCA results at Day 84 for 28 PD subjects given the drug product and 15 PD subjects given placebo (i.e. results from Visit 4). In treated group, there was a significant positive effect (p-value: 0.0001; log2foldchange: 0.27). There was also a significant effect in the group given placebo (p-value: 0.039; log2foldchange: 0.15). However, the effect was limited in the placebo group compared to the treated group.

Also, the present disclosure can present the ADAS-cog results at Day 28 for 47 AD subjects given the drug product and 22 AD subjects given placebo (i.e. results from Visit 3). In treated group, there was a significant positive effect (p-value: 0.000000322; log2foldchange: -0.32). There was also a (less) significant effect in the group given placebo (p-value: 0.009; log2foldchange: -0.15).

Further, the present disclosure can present the ADAS-cog results at Day 84 for 40 AD subjects given the drug product and 20 AD subjects given placebo (i.e. results from Visit 4). In treated group, there was a significant effect (p-value: 0.000013107; log2foldchange: -0.37). There was also a significant effect in the group given placebo (p-value: 0.001; log2foldchange: -0.19). However, the effect was limited in the placebo group compared to the treated group.

The differences between clinical parameters are analyzed by stratifying the patients into high- and low-scored ADAS-Cog groups (>20ADAS-Cog score is high, ≤20 is low). More interestingly, the present disclosure can present a significant difference of ADAS-Cog scores at day 28 in the high scored CMA group (p-value: 0.001; log2foldchange: -0.30) and no difference in the high scored placebo group (p-value: 0.08; log2foldchange: -0.1). Similarly, the present disclosure can present a significant difference of ADAS-Cog scores at day 84 in the high scored CMA group (p-value: 0.003; log2foldchange: -0.38) and no difference in the high scored placebo group (p-value: 0.3; log2foldchange: -0.07).

### ALZHEIMER'S DISEASE STUDY (RATS)

### Rat study design

12 weeks old female Sprague-Dawley rats (weighing 320-380g) were kept at a controlled temperature of 22 ± 2°C and a controlled humidity of 50 ± 5% on a 12-hour light/dark cycle. Food and water were available ad libitum. The animal experiments are performed at Medical Experimental Research Center, Ataturk University, Erzurum, Turkey. All experiments for the treatment of the animals were approved by the Ethics Committee of Atatürk University, and were conducted following the National Institutes of Health Guide for Care and Use of Laboratory Animals.

After acclimation to laboratory conditions for one (1) week, rats were randomly divided into two dietary regimens receiving either a Chow diet (CD) or a high-fat diet (HFD. The animals of Group 1 (n=4) were fed with only regular CD for five (5) weeks. The animals of Group 2 (n=4) were fed with CD and treated with STZ. The remaining animals (n=4) were fed with HFD for 3 weeks and sacrificed to verify the model development (Group 3). Groups 4-13 were fed with HFD for 5 weeks. After 3 weeks, the animals in Groups 5-13 were treated with STZ and administered with individual or combined substances. The animals in Group 4 were injected (icv) with a control substance (saline) after 3 weeks.

### Intracerebroventricular-streptozotocin (STZ) injection

Before surgical procedures, the rats were anesthetized by intraperitoneal administration of ketamine-xylazine (50 mg/kg ketamine and 5mg/kg xylazine) and placed individually in the stereotaxic instrument (Stoelting, Illinois, USA). Stereotaxic coordinates for injection were 0.8 mm posterior to the bregma, 1.5 mm lateral to the sagittal suture and 3.6 mm below the brain surface (Pellegrino et al. 1979). Then, 10 microliters of STZ (3 mg/kg, Sigma-Aldrich, Darmstadt, Germany) were injected over 3 min with a Hamilton micro-syringe into the bilateral ventricle. The injection needles were left in place for an additional 2 min to allow diffusion.

### Administration of substances

For carrying out the treatment experiments, the HFD rats were randomly separated into nine (9) subgroups of four (4) animals (Groups 5-13). As explained above, all animals of these groups had been treated with 3 mg/kg STZ (10 µL, icv).

The groups received the following treatments:
Group 5, control (no administration);
Group 6, serine (1000 mg/kg once daily by oral gavage);
Group 7, NAC (300 mg/kg once daily by oral gavage);
Group 8, L-carnitine tartrate (LCAT) (100 mg/kg once daily by oral gavage);
Group 9, nicotinamide riboside chloride (NRCl) (120 mg/kg once daily by oral gavage);
Group 10, nicotinamide (120 mg/kg once daily by oral gavage);
Group 11, serine (1000 mg/kg once daily by oral gavage), NAC (300 mg/kg once daily by oral gavage) and LCAT (100 mg/kg once daily by oral gavage);
Group 12, serine (1000 mg/kg once daily by oral gavage), NAC (300 mg/kg once daily by oral gavage), LCAT (100 mg/kg/day) and NRCl (120 mg/kg once daily by oral gavage);
Group 13, serine (1000 mg/kg once daily by oral gavage), NAC (300 mg/kg once daily by oral gavage), LCAT (100 mg/kg once daily by oral gavage) and nicotinamide (120 mg/kg once daily by oral gavage).

The body weight of the rats was recorded each week. After 5 weeks, all animals were anesthetized with isoflurane and sacrificed. Blood samples were collected from the abdominal aorta and centrifuged at 8000 rpm for 15 min at 4 °C. The plasma was separated and stored at -80 °C until further analysis. Other internal organs, including the heart, adipose tissues, liver, kidney, brain, muscle, intestine (duodenum, ileum, jejunum), pancreas, colon and stomach were immediately removed and then snap-frozen in liquid nitrogen and stored at -80°C.

### Tissue samples

Tissue samples obtained as a result of the experimental procedure were fixed in 10% buffered formalin solution for 48 hours. Following the routine tissue procedure, the tissues were embedded in paraffin blocks and 4 µm thick sections were taken from each block. Preparations prepared for histopathological examination were stained with hematoxylin-eosin (HE) and examined with a light microscope (Olympus BX51, Germany). According to histopathological findings, sections were evaluated as absent (-), very mild (+), mild (++), moderate (+++) and severe (++++).

### Immunohistochemical examination

Tissue sections taken on adhesive (poly-L-Lysin) slides for immunoperoxidase examination were deparaffinized and dehydrated. After washing the tissues with suppressed endogenous peroxidase activity in 3% H₂O₂ were boiled in antigen retrieval solution. In order to prevent nonspecific background staining in the sections, protein block compatible with all primary and secondary antibodies was dropped and incubated for 5 minutes. 8-OHdG (Cat no: sc-66036 dilution ratio: 1/100 US) was used as the primary antibody for brain tissues. 3-3' Diaminobenzidine (DAB) chromogen was used as chromogen, and according to their immunopositivity, sections were evaluated by independent pathologist and scored as absent (-), very mild (+), mild (++), moderate (+++) and severe (++++).

### Immunofluorescence examination

Tissue sections taken on adhesive (poly-L-Lysin) slides for immunoperoxidase examination were deparaffinized and dehydrated. After washing the tissues with suppressed endogenous peroxidase activity in 3% H₂O₂ were boiled in antigen retrieval solution. In order to prevent nonspecific background staining in the sections, protein block compatible with all primary and secondary antibodies was dropped and incubated for 5 minutes. For brain tissues, primary antibody Caspase 3 (Cat No:sc-56053 Dilution Ratio:1/100 US) was dropped and incubated for 1 hour at 37°C. After washing, secondary FITC (Cat No: ab6717 Dilution Ratio:1/500 UK) was dropped and incubated at 37°C for 30 minutes. The other primary antibody H2A.X (Cat No:I 0856-1 Dilution Ratio:1/100 US) was dropped and incubated at 37°C for 1 hour. After washing, secondary Texas Red (Cat No: sc-3917 Dilution Ratio:1/100 US) was dropped and incubated at 37°C for 30 minutes. DAPI (Cat No:D-1306 Dilution Ratio:1/200 US) was dripped onto the washed tissues and incubated in the dark for 5 minutes, then glycerin was sealed. Sections were examined under a fluorescence microscope (ZEISS Germany) by an independent pathologist and, according to their immunopositivity evaluated as absent (-), very mild (+), mild (++), moderate (+++) and severe (++++).

### Histopathological Findings (summarized in Table 1 below)

**Group 1 (chow reference group):** Brain tissues were found to be in normal histological structure. Brain tissues were evaluated as 8 OHdG negative in immunohistochemical staining, and Caspase-3 and H2A.X negative in immunofluorescence staining.

**Group 3 (HFD 3w reference group):** Moderate hyperemia in meningeal and parenchymal vessels, moderate degeneration and necrosis in neurons were observed in brain tissue. Immunohistochemical staining showed that moderate cytoplasmic 8 OHdG expression in brain tissues, while immunofluorescence staining showed moderate Caspase-3 and H2A.X expression in the brain.

**Group 4 (HFD 5w reference group):** Severe hyperemia in vessels, degeneration and necrosis in neurons were observed in brain tissues. Immunohistochemical staining of brain tissues showed severe cytoplasmic 8 OHdG expression and immunofluorescence staining of brain tissues showed severe cytoplasmic Caspase-3 and H2A.X expression.

**Group 2 (STZ reference group):** Moderate hyperemia in vessels, moderate degeneration and neurosis in neurons were detected in brain tissues. Moderate expression of 8 OHdG in immunohistochemical staining and moderate expression of Caspase-3 and H2A.X in immunfluorescence staining were determined in brain tissues.

**Group 5 (HFD+STZ reference group):** Severe hyperemia in brain tissues, severe degeneration and necrosis in neurons were detected. In brain tissues, severe cytoplasmic 8 OHdG expression in immunohistochemical staining was determined, and Caspase-3 and H2A.X expression in immunofluorescence staining were determined at a severe level.

**Group 6 (HFD+STZ+Serine reference group):** Moderate hyperemia, moderate degeneration and mild necrosis in neurons were detected in brain tissues. In brain tissues, 8 OHdG expression in immunohistochemical staining, Caspase-3 and H2A.X expression in immunofluorescence staining were found to be moderate.

**Group 7 (HFD+STZ+NAC reference group):** Severe hyperemia, severe degeneration of neurons and moderate necrosis were observed in brain tissues. In brain tissues, it was determined that 8 OHdG expression in immunohistochemical staining, Caspase-3 and H2A.X expression in immunofluorescence staining at moderate level.

**Group 8 (HFD+STZ+LCAT reference group):** Moderate hyperemia, moderate degeneration and mild necrosis in neurons were detected in brain tissues. In brain tissues, a moderate level of 8 OHdG expression was detected in immunohistochemical staining, a moderate level of Caspase-3 and H2A.X expressions were detected in immunofluorescence staining.

**Group 9 (HFD+STZ+NRCl reference group):** Moderate hyperemia, moderate degeneration and mild necrosis in neurons were detected in brain tissues. Immunohistochemical and immunofluorescence staining showed moderate expression of 8 OHdG, Caspase-3 and H2A.X in brain tissue.

**Group 10 (HFD+STZ+nicotinamide reference group):** Hyperemia, mild degeneration and very mild necrosis of neurons were detected in brain tissues. In brain tissues, were determined that moderate level of 8 OHdG expression in immunohistochemical staining, and mild Caspase-3 and H2A.X expression in immunofluorescence staining.

**Group 11 (HFD+STZ+serine+NAC+LCAT reference group):** Hyperemia in vessels, mild degeneration and very mild necrosis in neurons were detected in brain tissues. In brain tissues, were determined that mild 8 OHdG expression in immunohistochemical staining and mild Caspase-3 and H2A.X expression in immunofluorescence staining.

**Group 12 (HFD+STZ+serine+NAC+LCAT+NRCI reference group):** Hyperemia in vessels, mild degeneration and necrosis in neurons were detected in brain tissues. In brain tissues, immunohistochemical staining showed mild 8 OHdG expression, immunofluorescence staining showed mild Caspase-3 and H2A.X expressions.

**Group 13 (HFD+STZ+serine+NAC+LCAT+ nicotinamide group):** Hyperemia in vessels, very mild degeneration of neurons was detected in brain tissues. In brain tissues, very mild 8 OHdG expression was detected in immunohistochemical staining, and very mild Caspase-3 and H2A.X expressions were detected in immunofluorescence staining.

**Table 1: Scoring histopathological, immunohistochemical and immunofluorescence findings in brain tissues**

| **Group** | **Hyperemia** | **Degeneration in Neurons** | **Necrosis in Neurons** | **8-OHdG (IHC)** | **Caspase 3 (IFA FITC)** | **H2A.X (IFA Texas Red)** |
|---|---|---|---|---|---|---|
| **1** | - | - | - | - | - | - |
| **3** | +++ | +++ | +++ | +++ | +++ | +++ |
| **4** | ++++ | ++++ | ++++ | ++++ | ++++ | ++++ |
| **2** | +++ | +++ | +++ | +++ | +++ | +++ |
| **5** | ++++ | ++++ | ++++ | ++++ | ++++ | ++++ |
| **6** | +++ | +++ | ++ | +++ | +++ | +++ |
| **7** | ++++ | ++++ | +++ | +++ | +++ | +++ |
| **8** | +++ | +++ | ++ | +++ | +++ | +++ |
| **9** | +++ | +++ | ++ | +++ | +++ | +++ |
| **10** | ++ | ++ | + | +++ | ++ | ++ |
| **11** | ++ | ++ | ++ | ++ | ++ | ++ |
| **12** | ++ | ++ | + | ++ | ++ | ++ |
| **13** | ++ | + | - | + | + | + |

As shown in the table above, all substances had an effect when administrated alone (compare each of groups 6-10 to group 5). Nicotinamide was the most effective individual substance (compare group 10 to groups 6-9). Still, the effectiveness of nicotinamide alone is insufficient. However, when nicotinamide was given together with serine, NAC and LCAT, excellent results were obtained (see group 13). Notably, serine+NAC+LCAT+ nicotinamide gave much better results than serine+NAC+LCAT+NRCI (compare group 13 to group 12).

## Claims

1. A composition for use in a therapeutic method of treatment of a subject suffering from Alzheimer's disease or Parkinson's disease, said composition comprising:
A) serine;
B) N-acetyl cysteine, cysteine and/or cystine;
C) carnitine; and
D) nicotinamide.

2. The composition for use according to claim 1, wherein the molar ratio of A) to B) is between 16:1 and 1:4, such as between 12:1 and 1.5:1, preferably between 10:1 and 3:1.

3. The composition for use according to claim 1 or 2, wherein the molar ratio of A) to C) is between 150:1 and 1:1, such as between 100:1 and 2:1, preferably between 30:1 and 3:1, more preferably between 15:1 and 4:1.

4. The composition for use according to any one of the preceding claims, wherein the molar ratio of A) to D) is between 250:1 and 1.5:1, such as 150:1 and 3:1.

5. The composition for use according to any one of the preceding claims, wherein A) is L-serine.

6. The composition for use according to any one of the preceding claims, wherein B) is N-acetyl cysteine.

7. The composition for use according to any one of the preceding claims, which is powderous mixture.

8. The composition for use according to any one of the preceding claims, wherein the disease is Alzheimer's disease.

9. The composition for use according to any one of the preceding claims, wherein the subject is a human subject.

10. The composition for use according to any one of the preceding claims, wherein said therapeutic method comprises oral administration of composition.

11. The composition for use according to any one of the preceding claims, wherein said method comprises administration of:
A) in a dose of 0.48-24 mmol/kg/day, such as 0.48-4.8 mmol/kg/day, such as 1.8-4.8 mmol/kg/day, such as 2.9-4.7 mmol/kg/day;
B) in a dose of 0.31-3.05 mmol/kg/day, such as 0.31-1.84 mmol/kg/day, such as 0.40-1.23 mmol/kg/day;
C) in a dose of 0.100-2.50 mmol/kg/day, such as 0.200-2.00 mmol/kg/day, such as 0.230-1.00 mmol/kg/day, such as 0.300-0.800 mmol/kg/day; and
D) in a dose of 0.020-1.96 mmol/kg/day, such as 0.020-0.39 mmol/kg/day, such as 0.039-0.31 mmol/kg/day, such as 0.059-0.24 mmol/kg/day.

12. Substances comprising
A) serine,
B) N-acetyl cysteine, cysteine and/or cystine,
C) carnitine and
D) nicotinamide
for simultaneous, separate or sequential use in a method of treatment of a subject suffering from Alzheimer's disease or Parkinson's disease.

13. Substances for use according to claim 12, wherein said therapeutic method comprises oral administration of said substances.

14. Substances for use according to any one of claims 12-13, wherein said therapeutic method comprises oral administration of:
A) in a dose of 0.48-24 mmol/kg/day, such as 0.48-4.8 mmol/kg/day, such as 1.8-4.8 mmol/kg/day, such as 2.9-4.7 mmol/kg/day;
optionally B) in a dose of 0.31-3.05 mmol/kg/day, such as 0.31-1.84 mmol/kg/day, such as 0.40-1.23 mmol/kg/day;
optionally C) in a dose of 0.100-2.50 mmol/kg/day, such as 0.200-2.00 mmol/kg/day, such as 0.230-1.00 mmol/kg/day, such as 0.300-0.800 mmol/kg/day; and
D) in a dose of 0.020-1.96 mmol/kg/day, such as 0.020-0.39 mmol/kg/day, such as 0.039-0.31 mmol/kg/day, such as 0.059-0.24 mmol/kg/day.

## Patentansprüche

1. Zusammensetzung zur Verwendung bei einem therapeutischen Verfahren zur Behandlung eines Individuums, das an Alzheimer-Krankheit oder Parkinson-Krankheit leidet, wobei die Zusammensetzung Folgendes umfasst:
A) Serin;
B) N-Acetylcystein, Cystein und/oder Cystin;
C) Carnitin; und
D) Nicotinamid.

2. Zusammensetzung zur Verwendung nach Anspruch **1,** wobei das Molverhältnis von A) zu B) zwischen 16:1 und 1:4, wie zwischen 12:1 und 1,5:1, vorzugsweise zwischen 10:1 und 3:1, liegt.

3. Zusammensetzung zur Verwendung nach Anspruch 1 oder 2, wobei das Molverhältnis von A) zu C) zwischen 150:1 und 1:1, wie zwischen 100:1 und 2:1, vorzugsweise zwischen 30:1 und 3:1, weiter bevorzugt zwischen 15:1 und 4:1, liegt.

4. Zusammensetzung zur Verwendung nach einem der vorhergehenden Ansprüche, wobei das Molverhältnis von A) zu D) zwischen 250:1 und 1,5:1, wie 150:1 und 3:1, liegt.

5. Zusammensetzung zur Verwendung nach einem der vorhergehenden Ansprüche, wobei es sich bei A) um L-Serin handelt.

6. Zusammensetzung zur Verwendung nach einem der vorhergehenden Ansprüche, wobei es sich bei B) um N-Acetylcystein handelt.

7. Zusammensetzung zur Verwendung nach einem der vorhergehenden Ansprüche, bei der es sich um eine pulverförmige Mischung handelt.

8. Zusammensetzung zur Verwendung nach einem der vorhergehenden Ansprüche, wobei es sich bei der Krankheit um Alzheimer-Krankheit handelt.

9. Zusammensetzung zur Verwendung nach einem der vorhergehenden Ansprüche, wobei es sich bei dem Individuum um einen Menschen handelt.

10. Zusammensetzung zur Verwendung nach einem der vorhergehenden Ansprüche, wobei das therapeutische Verfahren die orale Verabreichung der Zusammensetzung umfasst.

11. Zusammensetzung zur Verwendung nach einem der vorhergehenden Ansprüche, wobei das Verfahren die Verabreichung von Folgendem umfasst:
A) in einer Dosis von 0,48-24 mmol/kg/Tag, wie 0,48-4,8 mmol/kg/Tag, wie 1,8-4,8 mmol/kg/Tag, wie 2,9-4,7 mmol/kg/Tag;
B) in einer Dosis von 0,31-3,05 mmol/kg/Tag, wie 0,31-1,84 mmol/kg/Tag, wie 0,40-1,23 mmol/kg/Tag;
C) in einer Dosis von 0,100-2,50 mmol/kg/Tag, wie 0,200-2,00 mmol/kg/Tag, wie 0,230-1,00 mmol/kg/Tag, wie 0,300-0,800 mmol/kg/Tag; und
D) in einer Dosis von 0,020-1,96 mmol/kg/Tag, wie 0,020-0,39 mmol/kg/Tag, wie 0,039-0,31 mmol/kg/Tag, wie 0,059-0,24 mmol/kg/Tag.

12. Substanzen, umfassend
A) Serin,
B) N-Acetylcystein, Cystein und/oder Cystin,
C) Carnitin und
D) Nicotinamid
zur gleichzeitigen, separaten oder aufeinanderfolgenden Anwendung bei einem Verfahren zur Behandlung eines Individuums, das an Alzheimer-Krankheit oder Parkinson-Krankheit leidet.

13. Substanzen zur Verwendung nach Anspruch 12, wobei das therapeutische Verfahren die orale Verabreichung der Substanzen umfasst.

14. Substanzen zur Verwendung nach einem der Ansprüche 12-13, wobei das therapeutische Verfahren die orale Verabreichung von Folgendem umfasst:
A) in einer Dosis von 0,48-24 mmol/kg/Tag, wie 0,48-4,8 mmol/kg/Tag, wie 1,8-4,8 mmol/kg/Tag, wie 2,9-4,7 mmol/kg/Tag;
gegebenenfalls B) in einer Dosis von 0,31-3,05 mmol/kg/Tag, wie 0,31-1,84 mmol/kg/Tag, wie 0,40-1,23 mmol/kg/Tag;
gegebenenfalls C) in einer Dosis von 0,100-2,50 mmol/kg/Tag, wie 0,200-2,00 mmol/kg/Tag, wie 0,230-1,00 mmol/kg/Tag, wie 0,300-0,800 mmol/kg/Tag; und
D) in einer Dosis von 0,020-1,96 mmol/kg/Tag, wie 0,020-0,39 mmol/kg/Tag, wie 0,039-0,31 mmol/kg/Tag, wie 0,059-0,24 mmol/kg/Tag.

## Revendications

1. Composition pour une utilisation dans un procédé thérapeutique de traitement d'un sujet souffrant de la maladie d'Alzheimer ou de la maladie de Parkinson, ladite composition comprenant :
A) sérine ;
B) N-acétylcystéine, cystéine et/ou cystine ;
C) carnitine ; et
D) nicotinamide.

2. Composition pour une utilisation selon la revendication 1, dans laquelle le rapport molaire de A) sur B) est compris entre 16:1 et 1:4, par exemple entre 12:1 et 1,5:1, de préférence entre 10:1 et 3:1.

3. Composition pour une utilisation selon la revendication 1 ou 2, dans laquelle le rapport molaire de A) sur C) est compris entre 150:1 et 1:1, par exemple entre 100:1 et 2:1, de préférence entre 30:1 et 3:1, plus préférablement entre 15:1 et 4:1.

4. Composition pour une utilisation selon l'une quelconque des revendications précédentes, dans laquelle le rapport molaire de A) sur D) est compris entre 250:1 et 1,5:1, par exemple 150:1 et 3:1.

5. Composition pour une utilisation selon l'une quelconque des revendications précédentes, dans laquelle A) est la L-sérine.

6. Composition pour une utilisation selon l'une quelconque des revendications précédentes, dans laquelle B) est la N-acétylcystéine.

7. Composition pour une utilisation selon l'une quelconque des revendications précédentes, qui est un mélange pulvérulent.

8. Composition pour une utilisation selon l'une quelconque des revendications précédentes, dans laquelle la maladie est la maladie d'Alzheimer.

9. Composition pour une utilisation selon l'une quelconque des revendications précédentes, dans laquelle le sujet est un sujet humain.

10. Composition pour une utilisation selon l'une quelconque des revendications précédentes, dans laquelle ledit procédé thérapeutique comprend l'administration orale de la composition.

11. Composition pour une utilisation selon l'une quelconque des revendications précédentes, dans laquelle ledit procédé comprend l'administration de :
A) en une dose de 0,48-24 mmol/kg/jour, telle que 0,48-4,8 mmol/kg/jour, telle que 1,8-4,8 mmol/kg/jour, telle que 2,9-4,7 mmol/kg/jour ;
B) en une dose de 0,31-3,05 mmol/kg/jour, telle que 0,31-1,84 mmol/kg/jour, telle que 0,40-1,23 mmol/kg/jour ;
C) en une dose de 0,100-2,50 mmol/kg/jour, telle que 0,200-2,00 mmol/kg/jour, telle que 0,230-1,00 mmol/kg/jour, telle que 0,300-0,800 mmol/kg/jour ; et
D) en une dose de 0,020-1,96 mmol/kg/jour, telle que 0,020-0,39 mmol/kg/jour, telle que 0,039-0,31 mmol/kg/jour, telle que 0,059-0,24 mmol/kg/jour.

12. Substances comprenant
A) sérine,
B) N-acétylcystéine, cystéine et/ou cystine,
C) carnitine et
D) nicotinamide
pour une utilisation simultanée, séparée ou séquentielle dans un procédé de traitement d'un sujet souffrant de la maladie d'Alzheimer ou de la maladie de Parkinson.

13. Substances pour une utilisation selon la revendication 12, dans lesquelles ledit procédé thérapeutique comprend l'administration orale desdites substances.

14. Substances pour une utilisation selon l'une quelconque des revendications 12 à 13, dans lesquelles ledit procédé thérapeutique comprend l'administration orale de :
A) en une dose de 0,48-24 mmol/kg/jour, telle que 0,48-4,8 mmol/kg/jour, telle que 1,8-4,8 mmol/kg/jour, telle que 2,9-4,7 mmol/kg/jour ;
éventuellement B) en une dose de 0,31-3,05 mmol/kg/jour, telle que 0,31-1,84 mmol/kg/jour, telle que 0,40-1,23 mmol/kg/jour ;
éventuellement C) en une dose de 0,100-2,50 mmol/kg/jour, telle que 0,200-2,00 mmol/kg/jour, telle que 0,230-1,00 mmol/kg/jour, telle que 0,300-0,800 mmol/kg/jour ; et
D) en une dose de 0,020-1,96 mmol/kg/jour, telle que 0,020-0,39 mmol/kg/jour, telle que 0,039-0,31 mmol/kg/jour, telle que 0,059-0,24 mmol/kg/jour.
